# EUROPEAN PATENT SPECIFICATION

(11) **EP 2 060 915 B1**
(45) Date of publication and mention of the grant of the patent: **17.08.2011**
(21) Application number: 07806826.9
(22) Date of filing: 06.09.2007
(51) Int. Cl.: G01N 33/53, G01N 33/543

(54) **METHOD FOR DETERMINATION OF ANTIGEN AND ANTIBODY AGAINST THE ANTIGEN**
VERFAHREN ZUR BESTIMMUNG VON ANTIGEN UND ANTIKÖRPER GEGEN DAS ANTIGEN
PROCÉDÉ DE DÉTERMINATION D'ANTIGÈNE ET D'ANTICORPS DIRIGÉ CONTRE L'ANTIGÈNE

(30) Priority: 08.09.2006 JP 2006244389
(43) Date of publication of application: 20.05.2009
(73) Proprietor: Nitto Boseki CO., LTD., Fukushima-shi, Fukushima 960-8161 (JP)
(72) Inventor: KOJIMA, Ryo, Koriyama-shi, Fukushima 963-0701 (JP); INOUE, Satoshi, Koriyama-shi, Fukushima 963-8025 (JP); ARAI, Satoshi, Ageo-shi, Saitama (JP); KATAYAMA, Katsuhiro, Koriyama-shi, Fukushima 963-8041 (JP); NODA, Kenta, Koriyama-shi, Fukushima 963-0702 (JP)
(74) Representative: HOFFMANN EITLE
(86) International application number: PCT/JP2007/067385
(87) International publication number: WO 2008/029873

(56) References cited:
- JP-A- 03 002 565
- JP-A- 04 048 265
- JP-A- 2001 504 572
- JP-A- 2005 172 546
- US-A- 4 210 622
- SALAMA A ET AL: "Rapid detection of antibodies to immunoglobulin A molecules by using the particle gel immunoassay" 20010701; 20010700, vol. 81, no. 1, 1 July 2001 (2001-07-01), pages 45-48, XP002351362
- DATABASE WPI Week 199125 Thomson Scientific, London, GB; AN 1991-181989 XP002541601 & JP 03 110470 A (TOA IYO-DENSHI KK) 10 May 1991 (1991-05-10)

## Description

### TECHNICAL FIELD

The present invention relates to a method for determination of an antigen and an antibody against the antigen . More particularly, the present invention relates to a method for determination of both of an antigen and an antibody against the antigen in a sample by an immuno-agglutination determination method by the use of one and the same reagent, for example, a method which permits determination of both of an antigen such as IgA and an antibody against the antigen, such as anti-IgA antibody by the use of one and the same reagent

### BACKGROUND ART

Determination of IgA in blood is like a routine in the clinical examination field because the level of IgA in blood is high in the case of chronic inflammation, chronic hepatitis, cirrhosis, IgA nephropathy, collagenosis (e.g. rheumatism), IgA type myeloma, etc. As a method for determining IgA, a method using immunoturbidimetry (TIA method) is usually adopted (patent document 1). TIA method is a method for determining an antigen such as IgA in a specimen which comprises subjecting the antigen in the specimen and an antibody as reagent to antigen-antibody reaction, and determining the amount of the antigen on the basis of the degree of the turbidity thus produced. This method is advantageous in that when an antigen (e.g. IgA) present in a high concentration is determined, the determination is possible without diluting a sample. This, method, however, is disadvantageous in that the cost of a reagent for the determination is high because a large amount of the antibody is used.

By the way, it is known that some people have almost no IgA and are deficient in IgA in some cases. It is considered that some of the people deficient in IgA have an antibody against IgA, i.e., anti-IgA antibody. It has been reported that if, by any chance, a person having anti-IgA antibody needs blood transfusion because of a disease or an accident, the transfusion of blood derived from a normal person, i.e., a person having IgA into the patient having anti-IgA antibody causes the antigen-antibody reaction between anti-IgA antibody and IgA in the blood of the patient, so that anaphylactic reaction, i.e., a shock is caused, resulting in a critical condition (non-patent document 2). Even the transfusion of blood derived from a normal person, i.e., a person having IgA into an IgA-deficient person having no anti-IgA antibody causes the production of anti-IgA antibody in the body, so that this person obtains the antibody. Therefore, such a person undergoes a shock reaction when the person receives the transfusion of blood derived from a normal person.

For preventing such a shock reaction, the blood of a person having no IgA is necessary as blood for transfusion to a patient having anti-IgA antibody. Therefore, it is necessary to investigate the presence of anti-IgA antibody in both of the bloods of a patient and a blood donor. Rapid detection of antibodies to immunoglobulin A. molecules can be achieved by using the particle gel immunoassay, where red high-density polystyrene beads are treated with purified IgA molecules (non-patent document 3).
Non-patent document 1: Nobuhiko Kubo et al., Nihon Rinshou, Vol. 57, Extra Number (1999), pp. 10-12
Non-patent document 2: Takako Migita et al., Nihon Yuketsu Gakkai-zasshi, Vol. 50, No. 3, pp. 419-424 (2004)
Non-patent document 3: A. Salama et al., Vox Sanguinis (2001) 81, 45-48

### DISCLOSURE OF THE INVENTION

### Problem to be Solved by the Invention

The present invention is intended to provide a method which make it possible to determine not only an antigen but also an antibody against the antigen in a sample by adopting a competitive homogeneous immuno-agglutination determination method which has been hardly adopted for determining an antigen or antibody in a sample, and a conventional immuno-agglutination determination method.

### Means for Solving the Problem

Under such a situation, the present inventor investigated a method for determining IgA in a sample. As a result, it was found that the purpose is achieved by the adoption of a competitive homogeneous immuno-agglutination determination method which has not often been adopted in the clinical examination field. It was also found that when the determination of IgA is attempted with a reagent used in this method, the value of IgA level becomes minus in some cases, depending on a specimen. Furthermore, it was found that such a specimen does not contain IgA and contains anti-IgA antibody. As a result, it was found that surprisingly, the reagent permits determination of not only IgA but also anti-IgA antibody in a sample. On the basis of this finding, the present inventor earnestly investigated in order to develop a method for determination of an antigen and an antibody in a sample at the same time by the use of one and the same reagent. The present invention has been accomplished in the course described above.

Therefore, the present invention relates to the items (1) to (6) described below.
(1) A method for determination of both an antigen and an antibody against the antigen in a sample containing an antigen and not containing an antibody against this antigen, or a sample not containing an antigen and containing an antibody against this antigen, or a sample containing neither an antigen nor an antibody against this antigen by the use of one and the same reagent, which comprises
   (i) using a determination reagent comprising an antibody capable of causing an antigen-antibody reaction with the antigen contained in the sample and an antigen capable of causing an antigen-antibody reaction with both the antibody contained in the sample and the antibody contained in the reagent, either the antigen or the antibody in the reagent being supported on microparticles,
   (ii) mixing the sample with the determination reagent, and
   (iii) determining the antigen or the antibody in the sample on the basis of the degree of increase or decrease in agglutination caused by the antigen-antibody reaction.
(2) A method according to item 1 which comprises
   (i) using a determination reagent comprising an antibody capable of causing an antigen-antibody reaction with the antigen contained in the sample and an antigen capable of causing an antigen-antibody reaction with both the antibody contained in the sample and the antibody contained in the reagent, the antigen in the reagent being supported on microparticles,
   (ii) mixing the sample with the determination reagent, and
   (iii) determining whether the sample contains antigen but not antibody or antibody but not antigen by
   (iv) a step, in the case where an antigen is contained in the sample of allowing the antigen contained in the sample and the antigen supported on the microparticles and constituting the determination reagent, to compete with each other for an antigen-antibody reaction with the antibody constituting the determination reagent, and determining the antigen in the sample on the basis of the degree of decrease in agglutination caused by the antigen-antibody reaction, or
   (v) a step, in the case where an antibody is contained in the sample, of subjecting the antigen supported on the microparticles and constituting the determination reagent to an antigen-antibody reaction with the antibody contained in the sample and the antibody constituting the determination reagent, and determining the antibody in the sample on the basis of the degree of increase in agglutination caused by the antigen-antibody reaction.
(3) A method according to item 1, which comprises
   (i) using a determination reagent comprising an antibody capable of causing an antigen-antibody reaction with the antigen contained in the sample and an antigen capable of causing an antigen-antibody reaction with both the antibody contained in the sample and the antibody contained in the reagent, the antibody in the reagent being supported on microparticles,
   (ii) mixing the sample with the determination reagent, and
   (iii) determining whether the sample contains antigen but not antibody or antibody but not antigen or neither an antigen nor an antibody by
   (iv) a step, in the case where an antigen is contained in the sample, of subjecting the antibody supported on the microparticles and constituting the determination reagent to an antigen-antibody reaction with the antigen contained in the sample and the antigen constituting the determination reagent, and determining the antigen in the sample on the basis of the degree of increase in agglutination caused by the antigen-antibody reaction, or
   (v) a step, in the case where an antibody is contained in the sample, of allowing the antibody contained in the sample and the antibody supported on the microparticles and constituting the determination reagent, to compete with each other for an antigen-antibody reaction with the antigen constituting the determination reagent, and determining the antibody in the sample on the basis of the degree of decrease in agglutination caused by the antigen-antibody reaction.
(4) A method according to any one of items 1 to 3, wherein the antigen in the sample is characterized in that it is usually present in biological samples derived from normal human beings or animals but is absent in biological samples derived from an extremely limited number of human beings or animals and that these human beings or animals have an antibody against the antigen.
(5) A method according to any one of items 1 to 4, wherein the antigen in the sample is IgA and the antibody in the sample is anti-IgA antibody.
(6) A method according to any one of items 1, 2, 4 and 5, which is practiced without dilution of the sample, by using a determination reagent comprising an antibody and an antigen supported on microparticles, to avoid prozone phenomenon.

### Advantages of the Invention

The following determination may be carried out by the adoption of a competitive homogeneous immuno-agglutination determination method and a conventional immuno-agglutination determination method by employing the determination method of the present invention. When an antigen is contained in a sample, it may be determined. When an antibody against the antigen is present in the sample, it is also possible to determine the antibody. In addition, the following may be carried out as one run of determination with one and the same reagent by using a small amount of an expensive antibody in this determination reagent. When an antigen but no antibody is present in a sample, it may be determined. When an antibody against the antigen but no antigen is present in the sample, it may be determined. Thus, both the antigen and the antibody - against the antigen in the sample may be determined. When neither an antigen nor an antibody against the antigen is present in a sample, it is also possible to confirm their absence by employing the determination method of the present invention.
When an antigen present in a high concentration in an ordinary biological sample, such as IgA is determined, the antigen in the sample may be determined without dilution of the sample, by using a determination reagent comprising an antibody against the antigen and an antigen supported on microparticles, to avoid prozone phenomenon.
Therefore, the determination method of the present invention may be employed for investigating the presence of anti-IgA antibody in both of the bloods of a blood donor and a recipient in order to prevent shock reaction at the time of blood transfusion. They may be used also in the field of clinical examinations for diagnosis of chronic inflammation, chronic hepatitis, cirrhosis, IgA nephropathy, collagenosis (e.g. rheumatism), IgA type myeloma or the like on the basis of IgA level.

### BEST MODE FOR CARRYING OUT THE INVENTION

In the present invention, the sample is, for example, a liquid sample derived from a living body. Specific examples of the sample are plasma, serum, urine, etc. Samples derived from blood, such as plasma and serum are suitable. In the present invention, as the sample, there are imagined three kinds of samples, i.e., a sample containing an antigen and not containing an antibody against this antigen, a sample not containing an antigen and containing an antibody against this antigen, and a sample containing neither an antigen nor an antibody against this antigen. Any of these samples may be subjected to the determination. In the case of a sample containing both of an antigen and an antibody against the antigen, antigen-antibody reaction occurs in a living body from which such a sample is collected, so that shock reaction occurs. Therefore, such a sample cannot exist.
As the antigen in the sample, an antigen suitable for the determination method of the present invention is "an antigen characterized in that it is usually present in biological samples derived from normal human beings or animals but is absent in biological samples derived from an extremely limited number of human beings or animals and that these human beings or animals have an antibody against the antigen". Such an antigen includes, for example, immunoglobulin A (IgA), haptoglobulin, α2-macroglobulin, complement C9 and complement C4.
As the antibody in the sample, an antibody suitable for the determination method of the present invention is an antibody against the above-exemplified antigen in the sample. Such an antibody includes, for example, anti-IgA antibody, anti-haptoglobulin antibody, anti-α2-macroglobulin antibody, anti-complement C4 antibody and anti-complement C4 antibody against the above-exemplified antigens, respectively.

The antibody in the reagent used in the determination method of the present invention is not particularly limited as long as it is capable of causing an antigen-antibody reaction with the antigen contained in the sample. When the antigen to be determined is IgA, haptoglobulin, α2-macroglobulin, complement C9 or complement C4, anti-IgA antibody, anti-haptoglobulin antibody, anti-α2-macroglobulin antibody, anti-complement C9 antibody and anti-complement C4 antibody, respectively, may be exemplified as the antibody in the reagent. As to the source of such an antibody, for example, when an antigen and an antibody against the antigen in a human sample are determined, the source may be any source as long as the antibody in the reagent is capable of causing an antigen-antibody reaction with the antigen contained in the sample. The antibody in the reagent includes, for example, antibodies derived from goat, antibodies derived from rabbit, and antibodies derived from rat. In addition, as the antibody in the reagent used in the present invention, either a monoclonal antibody or a polyclonal antibody may be used. Furthermore, a fragment of any antibody capable of causing an antigen-antibody reaction with the antigen contained in the sample may be used as the antibody contained in the reagent used in the present invention.
The antigen in the reagent used in the determination method of the present invention is not particularly limited as long as it is capable of causing an antigen-antibody reaction with both the antibody contained in the sample and the antibody contained in the reagent. When anti-IgA antibody, anti-haptoglobulin antibody, anti-α2-macroglobulin antibody, anti-complement C9 antibody or anti-complement C4 antibody is used as the antibody contained in the reagent, IgA, haptoglobulin, α2-macroglobulin, complement C9 and complement C4, respectively, may be exemplified as the antigen in the determination reagent. The source of the antigen in the reagent and a process for producing the antigen may be any source and any process as long as the antigen is capable of causing an antigen-antibody reaction with both the antibody contained in the sample and the antibody contained in the reagent. For example, when IgA or anti-IgA antibody in a human sample is determined, the antigen in the reagent includes, for example, IgA derived from goat, IgA derived from rabbit, IgA derived from rat, and IgA obtained by recombination. In addition, a fragment of any antigen capable of causing an antigen-antibody reaction with both the antibody contained in the sample and the antibody contained in the reagent may be used as the antigen contained in the reagent.
As the microparticles used in the determination method of the present invention, microparticles usually used in immuno-agglutination reaction may be used as they are. The most conventional microparticles are latex particles. As the microparticles, particles of 0.01 to 0.5 micron are usually used. As a method for supporting the antigen or the antibody on the microparticles used in the present invention, there may be adopted conventional supporting methods such as a physical adsorption method utilizing hydrophobic interaction, and a covalent bond method.

The determination method of the present invention is based on the fact that only one of the product and reactants in the immuno-agglutination reaction causes the appearance of a turbidity, while the other components are water-soluble or hardly cause the appearance of a turbidity. The principle of this fact is explained below by taking the case of using a determination reagent comprising an antibody and an antigen supported on microparticles.
At first, the reactants to be mixed are as follows: (i-1) an antigen derived from a sample, in the case where the antigen is present in the sample, (i-2) an antibody derived from the sample, in the case where the antibody is present in the sample, (ii) an antigen supported on microparticles and constituting a determination reagent, and (iii) an antibody constituting the determination reagent. All of these reactants (i) to (iii) are soluble or uniformly dispersible in water.

Then, the reactants are mixed to carry out an antigen-antibody reaction. When an antigen is present in the sample, there are competitively produced two antigen-antibody reaction products, i.e., an antigen-antibody reaction product of (iii) the antibody constituting the determination reagent with (i-1) the antigen derived from the sample and an antigen-antibody reaction product of (iii) the antibody constituting the determination reagent with (ii) the antigen supported on microparticles and constituting the determination reagent. The antigen-antibody reaction product of (iii) the antibody constituting the determination reagent with (ii) the antigen supported on microparticles and constituting the determination reagent is water-insoluble and causes the appearance of a turbidity, while the antigen-antibody reaction product of (iii) the antibody constituting the determination reagent with (i-1) the antigen derived from the sample hardly causes the appearance of a turbidity. Therefore, the turbidity of the reaction solution is increased with an increase in the amount of the antigen-antibody reaction product of (iii) the antibody constituting the determination reagent with (ii) the antigen supported on microparticles and constituting the determination reagent.

In the competitive reaction described above, (i-1) the antigen derived from the sample reacts with a definite amount of (iii) the antibody constituting the determination reagent, in competition with (ii) the antigen supported on microparticles and constituting the determination reagent, to reduce the amount of the insoluble antigen-antibody reaction product produced, i.e., the antigen-antibody reaction product of (iii) the antibody constituting the determination reagent with (ii) the antigen supported on microparticles and constituting the determination reagent and reduce the degree of turbidity produced in the reaction solution. Therefore, the turbidity of the reaction solution is reduced with an increase in the concentration of the antigen contained in the sample. Accordingly, the antigen in the sample may be determined on the basis of the degree of reduction of the turbidity.

When an antibody is present in the sample, antigen-antibody reaction products are produced by the reaction of each of (i-2) the antibody derived from the sample and (iii) the antibody constituting the determination reagent with (ii) the antigen supported on microparticles and constituting the determination reagent. Therefore, the amount of the antigen-antibody reaction product of (i-2) the antibody derived from the sample with (ii) the antigen supported on microparticles and constituting the determination reagent is increased with an increase in the concentration of the antibody contained in the sample, so that the degree of turbidity produced in the reaction solution is increased. Accordingly, the turbidity of the reaction solution is increased with an increase in the concentration of the antibody contained in the sample. Therefore, the antibody in the sample may be determined on the basis of the degree of increase of the turbidity.

Next, the principle is explained below by taking the case of using a determination reagent comprising an antigen and an antibody supported on microparticles.
At first, the reactants to be mixed are as follows: (i-1) an antigen derived from a sample, in the case where the antigen is present in the sample, (i-2) an antibody derived from the sample, in the case where the antibody is present in the sample, (ii) an antigen constituting a determination reagent, and (iii) an antibody supported on microparticles and constituting the determination reagent. All of these reactants (i) to (iii) are soluble or uniformly dispersible in water.

Then, the reactants are mixed to carry out an antigen-antibody reaction. When an antigen is present in the sample, there are produced two antigen-antibody reaction products, i.e., an antigen-antibody reaction product of (iii) the antibody supported on microparticles and constituting the determination reagent with (i-1) the antigen derived from the sample and an antigen-antibody reaction product of (iii) the antibody supported on microparticles and constituting the determination reagent with (ii) the antigen constituting the determination reagent. These antigen-antibody reaction products with the two antigens are water-insoluble and cause the appearance of a turbidity. Therefore, the amount of the antigen-antibody reaction product of (i-1) the antigen derived from the sample with (iii) the antibody supported on microparticles and constituting the determination reagent is increased with an increase in the concentration of the antigen contained in the sample, so that the degree of turbidity produced in the reaction solution is increased. Therefore, the turbidity of the reaction solution is increased with an increase in the concentration of the antigen contained in the sample. Accordingly, the antigen in the sample may be determined on the basis of the degree of increase of the turbidity.

When an antibody is present in the sample, there are competitively produced two antigen-antibody reaction products, i.e., an antigen-antibody reaction product of (ii) the antigen constituting the determination reagent with (i-2) the antibody derived from the sample and an antigen-antibody reaction product of (ii) the antigen constituting the determination reagent with (iii) the antibody supported on microparticles and constituting the determination reagent. In the competitive reaction described above, (i-2) the antibody derived from the sample reacts with (ii) the antigen constituting the determination reagent, in competition with a definite amount of (iii) the antibody supported on microparticles and constituting the determination reagent, to reduce the amount of the insoluble antigen-antibody reaction product produced, i.e., the antigen-antibody reaction product of (iii) the antibody supported on microparticles and constituting the determination reagent with (ii) the antigen constituting the determination reagent and reduce the degree of turbidity produced in the reaction solution. Therefore, the turbidity of the reaction solution is reduced with an increase in the concentration of the antibody contained in the sample. Accordingly, the antibody in the sample may be determined on the basis of the degree of reduction of the turbidity.

In the present invention, as to a method for measuring the degree of turbidity due to agglutination caused by the antigen-antibody reaction, the turbidity produced is usually measured by means of absorbance. The degree of turbidity is measurable also by visually observing a agglutination or by counting microparticles not agglutinated.

Specific examples of a method for practicing the determination method of the present invention are as follows.
When a reagent comprising an antibody and an antigen supported on microparticles is used as the determination reagent, the determination reagent is prepared at first by preparing a homogeneous dispersion of the antibody (e.g. anti-IgA antibody) in a buffer solution (e.g. phosphate buffer) (a first reagent) and a homogeneous dispersion of microparticles (e.g. latex particles) supporting thereon the same antigen (e.g. IgA) as an antigen to be determined (e.g. IgA) in a buffer solution (e.g. phosphate buffer) (a second reagent). Then, using an automatic analyzer (e.g. Autoanalyzer Hitachi Model 7180), an antigen-antibody reaction is carried out by adding the first reagent and then the second reagent to a sample containing the antigen or an antibody, which is to be determined, and the rate of agglutination caused is measured in terms of the degree of absorbance change by a two-point end method at a specific wavelength (for example, 340 nm to 800 nm). On the basis of the measured value thus obtained, the amount of the objective antigen or antibody in the sample may be determined by using a calibration curve previously obtained by the use of standard samples containing known concentrations of the antigen or the antibody.

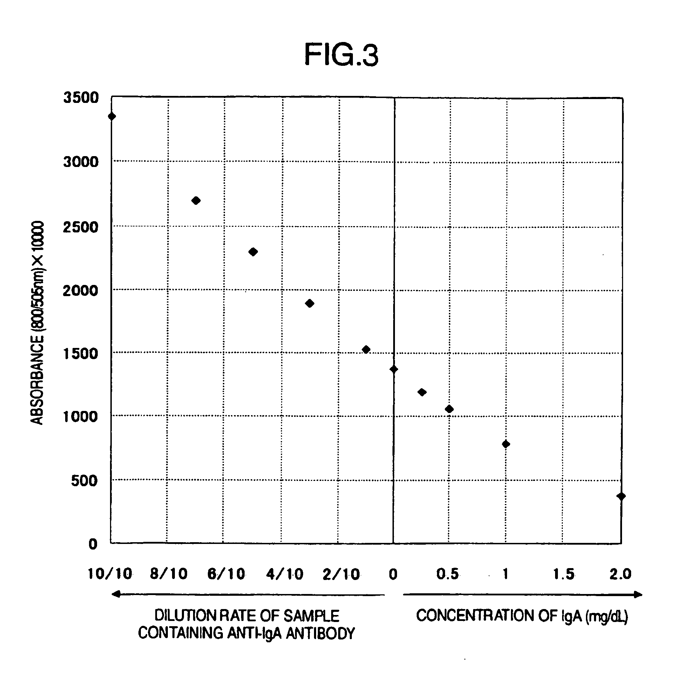

The calibration curve is suitably obtained as follows: as shown in Fig. 3 in Example 3 described hereinafter, the axis of abscissa refers to the antigen (e.g. IgA) concentration in its plus range and the antibody (e.g. anti-IgA antibody) concentration in its minus range, and the axis of ordinate refers to the degree of absorbance change. For example, in the case of the determination by the use of the calibration curve shown in Fig. 3, when the degree of change of measured absorbance (OD x 10000) is about 1400, i.e., an absorbance value at a position corresponding to a concentration of zero, namely, a position at which the calibration curve traverses the axis of ordinate, it may be judged that neither the antigen nor the antibody against the antigen is contained in the sample.

The using amounts of the antibody and the microparticles supporting the antigen thereon which constitute the determination reagent used for practicing the determination method are usually varied depending on the amounts of the antigen or the antibody to be determined and the sample used. Basically, the using amounts may be merely determined so that the calibration curve can be obtained by adopting a competitive homogeneous immuno-agglutination method using the determination reagent and that as a result of obtaining the calibration curve, the antigen can be determined. When IgA as antigen and anti-IgA antibody as antibody are determined with an automatic analyzer (e.g. Autoanalyzer Hitachi Model 7180), the using amounts are, for example, as follows.
When 1.5 to 35 µl, preferably 5 to 25 µl of the sample is used, the concentration of anti-IgA antibody contained in the first reagent is preferably adjusted so that its final concentration (the concentration in a mixture of the sample, the first reagent and the second reagent) may be 0.5 to 50 µg/ml (in terms of Becker titer), more preferably 1.5 to 15 µg/ml; the concentration of the IgA-sensitized latex contained in the second reagent is preferably adjusted to 0.005 to 0.5%, more preferably 0.015 to 0.15%; and the concentration of IgA used for the sensitization of latex (in terms of IgA weight) is preferably adjusted to 0.005 to 0.5 mg/ml, more preferably 0.015 to 0.15 mg/ml. In this case, the volume of each of the first reagent and the second reagent is preferably adjusted to 30 to 250 µl, more preferably 50 to 150 µl, and the total volume of the sample, the first reagent and the second reagent is preferably adjusted to 120 to 300 µl, more preferably 150 to 250 µl.

When a reagent comprising an antigen and an antibody supported on microparticles is used as the determination reagent, the determination reagent is prepared at first by preparing a homogeneous dispersion of the antigen in a buffer solution (e.g. Tris buffer) (a first reagent) and a homogeneous dispersion of microparticles (e.g. latex particles) supporting thereon an antibody against an antigen to be determined, in a buffer solution (e.g. Tris buffer) (a second reagent). Then, using an automatic analyzer (e.g. Autoanalyzer Hitachi Model 7180), an antigen-antibody reaction is carried out by adding the first reagent and then second reagent to a sample containing an antigen or an antibody, which is to be determined, and the rate of agglutination caused is measured in terms of the degree of absorbance change by a two-point end method at a specific wavelength (for example, 350 nm to 800 nm). On the basis of the measured value thus obtained, the amount of the objective antigen or the objective antibody in the sample may be determined by using a calibration curve previously obtained by the use of standard samples containing known concentrations of the antigen or the antibody.

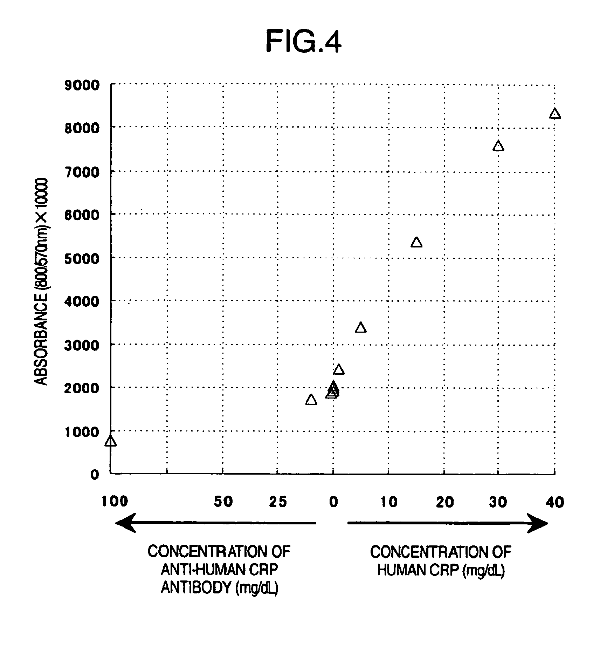

The calibration curve is suitably obtained as follows: as shown in Fig. 4 in Example 4 described hereinafter, the axis of abscissa refers to the antigen concentration in its plus range and the antibody concentration in its minus range, and the axis of ordinate refers to the degree of absorbance change. For example, in the case of the determination using the calibration curve shown in Fig. 4, when the degree of change of measured absorbance (OD x 10000) is about 2000, i.e., an absorbance value at a position corresponding to a concentration of zero, namely, a position at which the calibration curve traverses the axis of ordinate, it may be judged that neither the antigen nor the antibody against the antigen is contained in the sample.

The using amounts of the antigen and the microparticles supporting the antibody which constitute the determination reagent used for practicing the determination method are usually varied depending on the amounts of the antigen or the antibody to be determined and the sample used. Basically, the using amounts may be merely determined so that the calibration curve can be obtained by adopting a competitive homogeneous immuno-agglutination method using the determination reagent and that as a result of obtaining the calibration curve, the antigen can be determined. When the antigen and the antibody are determined with an automatic analyzer (e.g. Autoanalyzer Hitachi Model 7180), the using amounts are, for example, as follows.
When 1.5 to 35 µl, preferably 5 to 25 µl of the sample is used, the concentration of the antibody contained in the first reagent is preferably adjusted so that its final concentration (the concentration in a mixture of the sample, the first reagent and the second reagent) may be 0.5 to 50 µg/ml (in terms of Becker titer), more preferably 1.5 to 15 µg/ml; the concentration of the antibody-sensitized latex contained in the second reagent is preferably adjusted to 0.005 to 0.5%, more preferably 0.015 to 0.15%; and the concentration of the antibody used for the sensitization of latex is preferably adjusted to 0.05 to 10 mg/ml, more preferably 0.5 to 5 mg/ml. In this case, the volume of each of the first reagent and the second reagent is preferably adjusted to 30 to 250 µl, more preferably 50 to 150 µl, and the total volume of the sample, the first reagent and the second reagent is preferably adjusted to 120 to 300 µl, more preferably 150 to 250 µl.

As is clear from the above explanation, the determination reagent for practicing the determination method of the present invention comprises an antibody capable of causing an antigen-antibody reaction with an antigen contained in a sample and an antigen capable of causing an antigen-antibody reaction with both of an antibody contained in the sample and the antibody contained in the reagent, and either the antigen or the antibody in the reagent is supported on microparticles. In addition, if necessary, the determination reagent may contain conventional additives such as a diluting buffer solution, coating buffer solution, blocking reagent, preservative, stabilizer, etc.

The present invention is concretely illustrated with the following examples, which should not be construed as limiting the scope of the invention.

### Example 1

### Determination of human immunoglobulin A (IgA)

All of Examples 1 to 3 show a method for determining both of an antigen (IgA) and an antibody (anti-IgA antibody) by using a determination reagent comprising an antibody and an antigen supported on microparticles.

### 1) Preparation of IgA-sensitized latex particles (the antigen supported on microparticles)

IgA was adsorbed on latex particles as follows.

With 4 mL of a 1% solution of polystyrene latex particles with a particle diameter of 98 nm was mixed 4 mL of a solution obtained by dissolving human serum IgA in phosphate buffer to a concentration of 0.5 mg/mL, and the resulting mixture was stirred at room temperature for 1 hour. After the stirred mixture was centrifuged at 20,000 rpm for 45 minutes, the supernatant was disposed and the precipitate was recovered. To the precipitate was added 4 mL of a coating buffer solution to suspend the precipitate, and the resulting suspension was ultrasonicated to disperse the latex particles completely. The thus obtained human IgA-sensitized latex particles suspension having a latex concentration of 1% was kept in cold storage.

### 2) Preparation of a reagent for determining IgA

A first reagent and a second reagent were prepared as follows by using the latex particles having IgA adsorbed thereon and anti-human IgA antibody.

As the first reagent, a diluting buffer solution containing 0.2% of anti-human IgA goat serum (Becker titier: 8.8 mg/mL) was used.

As the second reagent, there was used a dilution obtained by diluting the human IgA-sensitized latex particles suspension having a latex concentration of 1% described in the above item 1) with diluting buffer solution.

The compositions of the reagents are as follows.

### Composition of the phosphate buffer

| | | |
|---|---|---|
| Sodium dihydrogenphosphate dihydrate | 20 mM | pH 7.50 |
| EDTA·2Na | 1 mM | |

### Composition of the coating buffer solution

| | | |
|---|---|---|
| Sodium dihydrogenphosphate dihydrate | 20 mM | pH 7.50 |
| EDTA·2Na | 1 mM | |
| Bovine serum albumin (BSA) | 1% | |
| Blocking reagent | 5% | |

### Composition of the diluting buffer solution

| | | |
|---|---|---|
| Sodium dihydrogenphosphate dihydrate | 20 mM | pH 7.50 |
| EDTA·2Na | 1 mM | |
| BSA | 1% | |
| Blocking reagent | 5% | |

### First reagent

| | | |
|---|---|---|
| Sodium dihydrogenphosphate dihydrate | 20 mM | pH 7.50 |
| EDTA·2Na | 1 mM | |
| BSA | 1% | |
| Blocking reagent | 5% | |
| Anti-human IgA goat serum | 0.2% | (v/v) |

### Composition of the second reagent

| | | |
|---|---|---|
| Sodium dihydrogenphosphate dihydrate | 20 mM | pH 7.50 |
| EDTA·2Na | 1 mM | |
| BSA | 1% | |
| Blocking reagent | 5% | |
| Human IgA-sensitized latex particles | 0.1% | (v/v) |

### 3) Calibration curve for determining IgA

As standard samples, there were used dilutions obtained by diluting serum having a known IgA concentration with the diluting buffer solution. The IgA concentration had been determined by the use of protein standard serum CRM470.

The standard samples were subjected to measurement with Autoanalyzer Hitachi Model 7170S by reacting 100 µL of the first reagent and 100 µL of the second reagent with 15 µL of serum as sample and measuring the degree of absorbance change by a two-point end method between the 19th and 26th photometric points (corresponding to a period between just after the addition of R2 and 2.5 minutes after the addition) at a dominant wavelength of 505 nm and a complementary wavelength of 800 nm.

### 4) Determination result

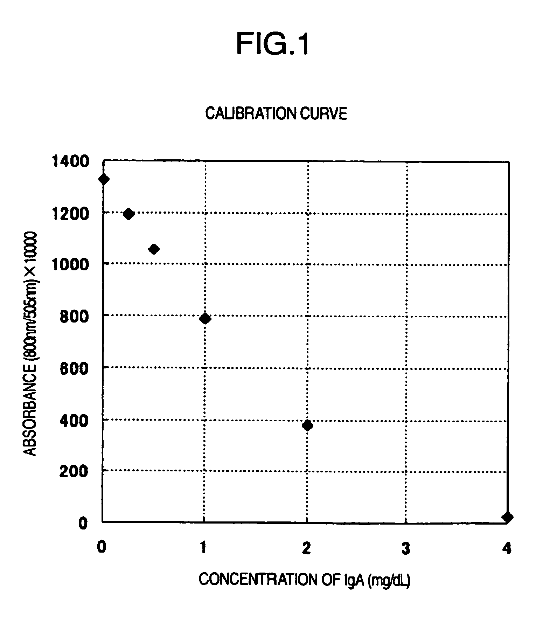

Table 1 shows the degree of absorbance change caused when the standard samples were subjected to the measurement by the use of the reagents described above, and Fig. 1 shows a graph (a calibration curve) showing the degree of absorbance change.

[Table 1]

**Table 1 Degree of absorbance change caused when the standard samples were subjected to the determination**

| Concentration of serum IgA of (mg/dL) | Degree of absorbance change (OD × 10000) |
|---|---|
| | Dominant wavelength 505 nm, Complementary wavelength 800 nm |
| 0 | 1324 |
| 0.25 | 1195 |
| 0.5 | 1058 |
| 1.0 | 788 |
| 2.0 | 376 |
| 4.0 | 24 |

As shown in Table 1 and Fig. 1, absorbance is decreased with an increase in the IgA concentration in the samples. That is, as the determination method of the present invention, the method described above utilizes the fact that after the reaction of IgA contained as antigen in the sample with anti-human IgA goat antibody contained in the first reagent, anti-human IgA goat antibody remaining after the reaction reacts with the IgA-sensitized latex particles contained in the second reagent. In other words, when the IgA concentration in the sample is low, anti-IgA antibody in the first reagent remains in a large amount and reacts with the IgA-sensitized latex particles contained in the second reagent, to a large extent. However, since the amount of anti-IgA antibody remaining in the first reagent is decreased with an increase in the IgA concentration in the sample, the extent of the reaction of anti-IgA antibody with the IgA-sensitized latex contained in the second reagent is diminished, resulting in a low absorbance value.

### Example 2

### Correlation between the determination method of the present invention and TIA method

### 1) Preparation of an IgA determination reagent and determination conditions

The same reagents and determination conditions as in Example 1 were employed.

### 2) Quantitation with an automatic analyzer

Quantitation was carried out by using the standard samples described in Example 1 and the function of giving a multipoint calibration curve of Autoanalyzer Hitachi Model 7170S.

### 3) Determination in serum samples

As samples, dilutions obtained by properly diluting serum with the diluting buffer solution were used. The correlation was confirmed by using "N-assay TIA IgA-SH" (Nitto Boseki Co., Ltd.), a commercial reagent for TIA method, as a reference reagent. TIA method was practiced according to specified parameters by similarly using Autoanalyzer Hitachi Model 7170S.

### 4) Determination result

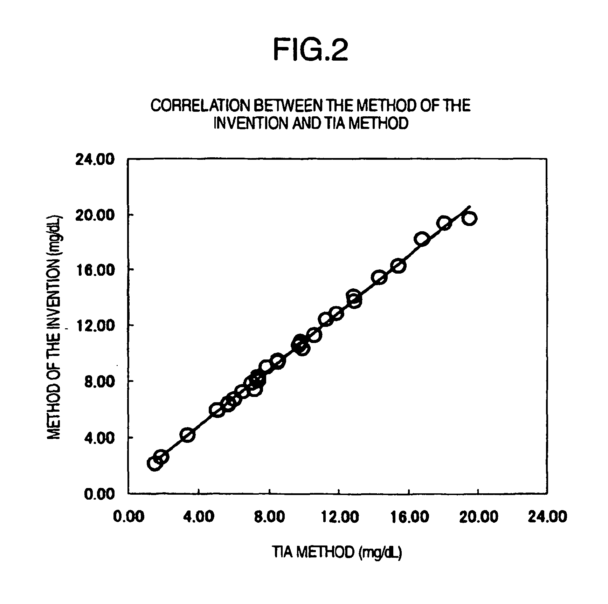

Fig. 2 shows the result of investigating the correlation between the determination method of the present invention and TIA method. As shown in Fig. 2, the correlation was confirmed by taking TIA method as X and the method of the present invention as Y, to obtain the following good result: Y = 1.02X + 0.7; coefficient of correlation 0.998 (N = 30). This result indicates that the method of the present invention permits accurate determination of the IgA concentration in serum.

### Example 3

### Determination of IgA and anti-IgA antibody

### 1) Preparation of a determination reagent and determination conditions

The same reagents and determination conditions as in Example 1 were employed.

### 2) Samples for determination

As samples, there were used dilutions obtained by proper serial dilution of a specimen containing IgA or anti-IgA antibody with the diluting buffer solution.

### 3) Quantitation with an automatic analyzer

Quantitation was carried out by using the standard samples of 0 mg/ml and 1 mg/ml among the standard samples described in Example 1 and the function of giving two-point calibration curve of Autoanalyzer Hitachi Model 7170S.

### 4) Determination result

Table 2 and Fig. 3 show the result of determining IgA and anti-IgA antibody in the samples.

[Table 2]

**Table 2 The result of determining IgA and anti-IgA antibody in the samples**

| Sample | | Degree of absorbance change (OD × 10000) |
|---|---|---|
| IgA concentration (mg/dL) | Dilution rate of sample containing anti-IgA antibody | Dominant wavelength 505 nm Complementary wavelength 800 nm |
| 2.0 | - | 376 |
| 1.0 | - | 788 |
| 0.5 | - | 1058 |
| 0.25 | - | 1195 |
| 0.0 | 0/10 | 1380 |
| 0.0 | 1/10 | 1526 |
| 0.0 | 3/10 | 1894 |
| 0.0 | 5/10 | 2295 |
| 0.0 | 7/10 | 2701 |
| 0.0 | 10/10 | 3345 |

As shown in Table 2 and Fig. 3, the result of determining IgA and anti-IgA antibody in the samples obtained by the serial dilution showed a satisfactory linear relationship in the areas on both sides of the origin. This fact indicates the following: in the determination method of the present invention, the IgA concentration in a sample is measured by the use of the origin and a calibration curve obtained by using standard samples containing known concentrations of IgA, and moreover, when anti-IgA antibody is present in the sample, the anti-IgA antibody concentration is defined as a minus IgA concentration value and is accurately measured.

### Example 4

### Determination of human CRP and anti-human CRP antibody

It was confirmed that both of an antigen and an antibody may be determined by using a determination reagent comprising an antigen and an antibody supported on microparticles.

Specifically, the concentration of human CRP (an antigen) or anti-human CRP antibody (an antibody) in a specimen was measured by the use of a determination reagent comprising a latex sensitized by anti-human CRP antibody (an antibody supported on microparticles) and human CRP (an antigen). When the specimen contained anti-human CRP antibody, a specimen containing anti-human CRP antibody derived from goat was used as a model sample.

### 1) Preparation of latex particles sensitized by anti-human CRP antibody

Anti-human CRP antibody was adsorbed on latex particles as follows.

With 100 mL of a 1% solution of polystyrene latex particles with a particle diameter of 120 nm was mixed 100 mL of a solution obtained by dissolving anti-human CRP antibody in Tris buffer to a concentration of 3.0 mg/mL, and the resulting mixture was stirred at room temperature for 1 hour. After the stirred mixture was centrifuged at 20,000 rpm for 45 minutes, the supernatant was disposed and the precipitate was recovered. To the precipitate was added 100 mL of a coating buffer solution to suspend the precipitate, and the resulting suspension was ultrasonicated to disperse the latex particles completely, and then was stirred at room temperature for 1 hour. Thereafter, the stirred suspension was centrifuged and to the resulting precipitate was added 100 mL of Tris buffer to suspend the precipitate. The resulting suspension was ultrasonicated to disperse the latex particles completely, whereby a 1% suspension of latex particles sensitized by anti-human CRP antibody was obtained.

### 2) Preparation of a reagent for determination of human CRP and anti-human CRP antibody

A first reagent and a second reagent were prepared as follows by the use of the latex particles sensitized by anti-human CRP antibody and human CRP.

As the first reagent, Tris buffer containing 0.10 mg/dL of human CRP was used.

As the second reagent, there was used a dilution obtained by 5-fold dilution of the 1% suspension of latex particles sensitized by anti-human CRP antibody described in the above item 1) with Tris buffer.

The compositions of the reagents are as follows.

### Composition of the Tris buffer

| | | |
|---|---|---|
| Tris (tris(hydroxymethyl)aminoethane) | 50 mM | pH 7.5 |
| Sodium chloride | 150 mM | |

### Composition of a coating buffer solution

| | | |
|---|---|---|
| Tris | 50 mM | pH 7.5 |
| Sodium chloride | 150 mM | |
| BSA (bovine serum albumin) | 1.0% | |

### First reagent

| | |
|---|---|
| Tris buffer | |
| Human CRP | 0.10 mg/dL |

### Second reagent

| | |
|---|---|
| Tris buffer | |
| The latex particles sensitized by | |
| anti-human CRP antibody | 0.2% (v/v) |

### 3) Determination method

The determination was carried out with Autoanalyzer Hitachi Model 7180 by reacting 120 µL of the first reagent and 120 µL of the second reagent with 2.4 µL of each of samples and measuring the degree of absorbance change by a two-point end method between the 18th and 28th photometric points (corresponding to a period between just after the addition of R2 and 2.9 minutes after the addition) at a dominant wavelength of 570 nm and a complementary wavelength of 800 nm.

As the samples, there were used dilutions obtained by properly diluting a specimen containing human CRP and anti-human CRP antibody with physiological saline.

### 4) Determination result

Table 3 and Fig. 4 show the result of determining human CRP and anti-human CRP antibody in the samples by the use of the reagents described above.

[Table 3]

**Table 3 The result of determining human CRP and anti-human CRP antibody in the samples**

| Sample | | Degree of absorbance change (OD × 10000) |
|---|---|---|
| Concentration of human CRP (mg/dL) | Concentration of anti-human CRP antibody (mg/dL) | Dominant wavelength 570 nm Complementary wavelength 800 nm |
| 40 | - | 8382 |
| 30 | - | 7617 |
| 15 | - | 5384 |
| 5 | - | 3408 |
| 1 | - | 2436 |
| 0 | - | 2048 |
| - | 0.01 | 2000 |
| - | 0.10 | 1935 |
| - | 1.00 | 1893 |
| - | 10.00 | 1749 |
| - | 100.00 | 775 |

As can be seen from the result of determining human CRP and anti-human CRP antibody in the samples which is shown in Table 3 and Fig. 4, absorbance was increased with an increase of the human CRP concentration in the samples, and was decreased with an increase of the anti-human CRP antibody concentration in the samples. This fact means the following: according to the method of the present invention, by the employment of a calibration curve obtained by the use of a standard sample containing 0 mg/dL of human CRP and standard samples containing known concentrations of human CRP, the human CRP concentration in the samples may be measured and moreover, when anti-human CRP antibody is present in the samples, the anti-human CRP antibody concentration may be measured as a minus human CRP concentration value.

### INDUSTRIAL APPLICABILITY

By employing the determination method of the present invention, an antigen in a sample may be determined by the use of an immuno-agglutination determination system and when an antibody against the antigen is present in the sample, it is also possible to determine the antibody by the use of the same reagent as for the antigen. In addition, the following may be carried out as one run of determination with one and the same reagent by using a small amount of an expensive antibody in the determination reagent. When an antigen but no antibody is present in a sample, it may be determined. When an antibody against the antigen but no antigen is present in the sample, it may be determined. Thus, both the antigen and the antibody against the antigen in the sample may be determined. When neither an antigen nor an antibody against the antigen is present in a sample, it is also possible to confirm their absence by employing the determination method of the present invention.

### BRIEF DESCRIPTION OF DRAWINGS

[Fig. 1] A graph showing the degree of absorbance change in the case of subjecting standard samples to determination in Example 1.
[Fig. 2] A graph showing the correlation between the determination method of the present invention and TIA method.
[Fig. 3] A graph showing the result of determining IgA and anti-IgA antibody in samples in Example 3.
[Fig. 4] A graph showing the result of determining CRP and anti-CRP antibody in samples in Example 4.

## Claims

1. A method for determination of both ran antigen and an antibody against the antigen in a sample containing an antigen and not containing an antibody against this antigen, or a sample not containing an antigen and containing an antibody against this antigen, or a sample containing neither an antigen nor an antibody against this antigen by the use of one and the same reagent, which comprises
(i) using a determination reagent comprising an antibody capable of causing an antigen-antibody reaction with the antigen contained in the sample and an antigen capable of causing an antigen-antibody reaction with both the antibody contained in the sample and the antibody contained in the reagent, either the antigen or the antibody in the reagent being supported on microparticles,
(ii) mixing the sample with the determination reagent, and
(iii) determining the antigen or the antibody in the sample on the basis of the degree of increase or decrease in agglutination caused by the antigen-antibody reaction.

2. A method according to claim 1, which comprises
(i) using a determination reagent comprising an antibody capable of causing an antigen-antibody reaction with the antigen contained in the sample and an antigen capable of causing an antigen-antibody reaction with both the antibody contained in the sample and the antibody contained in the reagent, the antigen in the reagent being supported on microparticles,
(ii) mixing the sample with the determination reagent, and
(iii) determining whether the sample contains antigen but not antibody or antibody but not antigen by
(iv) a step, in the case where an antigen is contained in the sample, of allowing the antigen contained in the sample and the antigen supported on the microparticles and constituting the determination reagent, to compete with each other for an antigen-antibody reaction with the antibody constituting the determination reagent, and determining the antigen in the sample on the basis of the degree of decrease in agglutination caused by the antigen-antibody reaction, or
(v) a step, in the case where an antibody is contained in the sample, of subjecting the antigen supported on the microparticles and constituting the determination reagent to an antigen-antibody reaction with the antibody contained in the sample and the antibody constituting the determination reagent, and determining the antibody in the sample on the basis of the degree of increase in agglutination caused by the antigen-antibody reaction.

3. A method according to claim 1, which comprises
(i) using a determination reagent comprising an antibody capable of causing an antigen-antibody reaction with the antigen contained in the sample and an antigen capable of causing an antigen-antibody reaction with both the antibody contained in the sample and the antibody contained in the reagent, the antibody in the reagent being supported on microparticles,
(ii) mixing the sample with the determination reagent, and
(iii) determining whether the sample contains antigen but not antibody or antibody but not antigen or neither an antigen nor an antibody by
(iv) a step, in the case where an antigen is contained in the sample, of subjecting the antibody supported on the microparticles and constituting the determination reagent to an antigen-antibody reaction with the antigen contained in the sample and the antigen constituting the determination reagent, and determining the antigen in the sample on the basis of the degree of increase in agglutination caused by the antigen-antibody reaction, or
(v) a step, in the case where an antibody is contained in the sample, of allowing the antibody contained in the sample and the antibody supported on the microparticles and constituting the determination reagent, to compete with each other for an antigen-antibody reaction with the antigen constituting the determination reagent, and determining the antibody in the sample on the basis of the degree of decrease in agglutination caused by the antigen-antibody reaction.

4. A method according to any one of claims 1 to 3, wherein the antigen in the sample is **characterized in that** it is usually present in biological samples derived from normal human beings or animals but is absent in biological samples derived from an extremely limited number of human beings or animals and that these human beings or animals have an antibody against the antigen.

5. A method according to any one of claims 1 to 4, wherein the antigen in the sample is IgA and the antibody in the sample is anti-IgA antibody.

6. A method according to any one of claims 1, 2, 4 and 5, which is practiced without dilution of the sample, by using a determination reagent comprising an antibody and an antigen supported on microparticles, to avoid prozone phenomenon.

## Patentansprüche

1. Verfahren zur Bestimmung sowohl eines Antigens als auch eines Antikörpers gegen das Antigen in einer Probe, die das Antigen, aber nicht den Antikörper gegen dieses Antigen enthält, oder einer Probe, die nicht ein Antigen, aber einen Antikörper gegen dieses Antigen enthält, oder einer Probe, die weder ein Antigen noch einen Antikörper gegen dieses Antigen enthält unter Verwendung von einem und demselben Reagens, das umfasst
(i) das Verwenden eines Bestimmungsreagens, umfassend einen Antikörper, der befähigt ist, eine Antigen-Antikörper-Reaktion mit dem Antigen, das in der Probe enthalten ist, einzugehen, sowie ein Antigen, das befähigt ist, eine Antigen-Antikörper-Reaktion mit dem Antiköper, der in der Probe enthalten ist, und dem Antikörper, der in dem Reagens enthalten ist, einzugehen, wobei entweder das Antigen oder der Antikörper in dem Reagens auf Mikropartikeln geträgert ist,
(ii) das Mischen der Probe mit dem Bestimmungsreagens, und
(iii) das Bestimmen des Antigens oder des Antikörpers in der Probe auf Basis des Grades der Zunahme oder Abnahme der Agglutination, die durch die Antigen-Antikörper-Reaktion verursacht wird.

2. Verfahren gemäß Anspruch 1, das umfasst
(i) das Verwenden eines Bestimmungsreagens, umfassend einen Antikörper, der befähigt ist, eine Antigen-Antikörper-Reaktion mit dem Antigen, das in der Probe enthalten ist, einzugehen, sowie ein Antigen, das befähigt ist, eine Antigen-Antikörper-Reaktion mit dem Antiköper, der in der Probe enthalten ist, und dem Antikörper, der in dem Reagens enthalten ist, einzugehen, wobei entweder das Antigen oder der Antikörper in dem Reagens auf Mikropartikeln geträgert ist,
(ii) das Mischen der Probe mit dem Bestimmungsreagens, und
(iii) das Bestimmen, ob die Probe Antigen, aber keinen Antikörper, oder Antikörper, aber kein Antigen, enthält, durch
(iv) einen Schritt, im Fall, dass ein Antigen in der Probe enthalten ist, bei dem ermöglicht wird, dass das in der Probe enthaltene Antigen und das auf den Mikropartikeln geträgerte Antigen, das das Bestimmungsreagens bildet, miteinander um eine Antigen-Antikörper-Reaktion mit dem Antikörper, der das Bestimmungsreagens bildet, konkurrieren, sowie das Bestimmen des Antigens in der Probe auf Basis des Grades der Abnahme der Agglutination, die durch die Antigen-Antikörper-Reaktion verursacht wird, oder
(v) einen Schritt, im Fall, dass ein Antikörper in der Probe enthalten ist, bei dem das auf den Mikropartikeln geträgerte Antigen, das das Bestimmungsreagens bildet, eine Antigen-Antikörper-Reaktion mit dem in der Probe enthaltenen Antikörper und dem Antikörper, der das Bestimmungsreagens bildet, unterzogen wird, sowie Bestimmen des Antikörpers in der Probe auf Basis des Grades der Zunahme der Agglutination, die durch die Antigen-Antikörper-Reaktion verursacht wird.

3. Verfahren gemäß Anspruch 1, das umfasst
(i) das Verwenden eines Bestimmungsreagens, umfassend einen Antikörper, der befähigt ist, eine Antigen-Antikörper-Reaktion mit dem Antigen, das in der Probe enthalten ist, einzugehen, sowie ein Antigen, das befähigt ist eine Antigen-Antikörper-Reaktion mit dem Antiköper, der in der Probe enthalten ist und dem Antikörper, der in dem Reagens enthalten ist, einzugehen, wobei entweder das Antigen oder der Antikörper in dem Reagens auf Mikropartikeln geträgert ist,
(ii) das Mischen der Probe mit dem Bestimmungsreagens, und
(iii) das Bestimmen, ob die Probe Antigen, aber nicht Antikörper, oder Antikörper, aber nicht Antigen, oder weder ein Antigen noch einen Antikörper enthält, durch
(iv) einen Schritt, im Fall, dass ein Antigen in der Probe enthalten ist, bei dem der Antikörper, der auf den Mikropartikeln geträgert ist und der das Bestimmungsreagens bildet, einer Antigen-Antikörper-Reaktion mit dem Antigen, das in der Probe enthalten ist, und dem Antigen, das das Bestimmungsreagens bildet, unterzogen wird, sowie das Bestimmen des Antigens in der Probe auf Basis des Grades der Zunahme der Agglutination, die durch die Antigen-Antikörper-Reaktion verursacht wird, oder
(v) einen Schritt, im Fall, dass ein Antikörper in der Probe enthalten ist, bei dem ermöglicht wird, dass der in der Probe enthaltene Antikörper und der auf den Mikropartikeln geträgerte Antikörper, der das Bestimmungsreagens bildet, miteinander um eine Antigen-Antikörper-Reaktion mit dem Antigen konkurrieren, das das Bestimmungsreagens bildet, und Bestimmen des Antikörpers in der Probe auf Basis des Grades der Abnahme der Agglutination, die durch die Antigen-Antikörper-Reaktion verursacht wird.

4. Verfahren gemäß einem der Ansprüche 1 bis 3, wobei das Antigen in der Probe **dadurch** charakterisiert ist, dass es normalerweise in biologischen Proben, die von normalen Menschen oder Tieren abgeleitet sind, vorhanden ist, aber in biologischen Proben, die von einer stark eingeschränkten Anzahl von Menschen oder Tieren abgeleitet sind, abwesend ist, und wobei diese Menschen oder Tiere einen Antikörper gegen das Antigen haben.

5. Verfahren gemäß einem beliebigen der Ansprüche 1 bis 4, wobei das Antigen in der Probe IgA und der Antikörper in der Probe ein Anti-IgA-Antikörper ist.

6. Verfahren gemäß einem beliebigen der Ansprüche 1, 2, 4 und 5, das ohne Verdünnung der Probe ausgeführt wird durch Verwendung eines Bestimmungsreagens, das einen Antikörper und ein auf Mikropartikeln geträgertes Antigen umfasst, um das Prozonen-Phänomen zu vermeiden.

## Revendications

1. Procédé pour déterminer à la fois un antigène et un anticorps dirigé contre l'antigène dans un échantillon contenant un antigène et ne contenant pas d'anticorps dirigé contre cet antigène, ou un échantillon ne contenant pas d'antigène et contenant un anticorps dirigé contre cet antigène, ou un échantillon ne contenant ni antigène ni anticorps dirigé contre cet antigène, en utilisant un seul et même réactif, qui comprend
(i) utiliser un réactif de détermination comprenant un anticorps capable de provoquer une réaction antigène-anticorps avec l'antigène contenu dans l'échantillon et un antigène capable de provoquer une réaction antigène-anticorps avec à la fois l'anticorps contenu dans l'échantillon et l'anticorps contenu dans le réactif, l'antigène ou l'anticorps dans le réactif étant fixé sur des microparticules,
(ii) mélanger l'échantillon avec le réactif de détermination, et
(iii) déterminer l'antigène et l'anticorps dans l'échantillon en se basant sur le degré d'augmentation ou de diminution de l'agglutination provoquée par la réaction antigène-anticorps.

2. Procédé selon la revendication 1, qui comprend
(i) utiliser un réactif de détermination comprenant un anticorps capable de provoquer une réaction antigène-anticorps avec l'antigène contenu dans l'échantillon et un antigène capable de provoquer une réaction antigène-anticorps avec à la fois l'anticorps contenu dans l'échantillon et l'anticorps contenu dans le réactif, l'antigène dans le réactif étant fixé sur des microparticules,
(ii) mélanger l'échantillon avec le réactif de détermination, et
(iii) déterminer si l'échantillon contient un antigène mais pas d'anticorps, ou un anticorps mais pas d'antigène, par
(iv) une étape, dans le cas où un antigène est contenu dans l'échantillon, qui consiste à permettre à l'antigène contenu dans l'échantillon et à l'antigène fixé sur les microparticules, constituant le réactif de détermination, d'être en compétition l'un avec l'autre pour une réaction antigène-anticorps avec l'anticorps constituant le réactif de détermination, et à déterminer l'antigène dans l'échantillon en se basant sur le degré de diminution de l'agglutination provoquée par la réaction antigène-anticorps, ou
(v) une étape, dans le cas où un anticorps est contenu dans l'échantillon, qui consiste à soumettre l'antigène fixé sur les microparticules, constituant le réactif de détermination, à une réaction antigène-anticorps avec l'anticorps contenu dans l'échantillon et l'anticorps constituant le réactif de détermination, et à déterminer l'anticorps dans l'échantillon en se basant sur le degré d'augmentation de l'agglutination provoquée par la réaction antigène-anticorps.

3. Procédé selon la revendication 1, qui comprend
(i) utiliser un réactif de détermination comprenant un anticorps capable de provoquer une réaction antigène-anticorps avec l'antigène contenu dans l'échantillon et un antigène capable de provoquer une réaction antigène-anticorps avec à la fois l'anticorps contenu dans l'échantillon et l'anticorps contenu dans le réactif, l'anticorps dans le réactif étant fixé sur des microparticules,
(ii) mélanger l'échantillon avec le réactif de détermination, et
(iii) déterminer si l'échantillon contient un antigène mais pas d'anticorps, ou un anticorps mais pas d'antigène, ou ni antigène ni anticorps, par
(iv) une étape, dans le cas où un antigène est contenu dans l'échantillon, qui consiste à soumettre l'anticorps fixé sur les microparticules, constituant le réactif de détermination, à une réaction antigène-anticorps avec l'antigène contenu dans l'échantillon et l'antigène constituant le réactif de détermination, et à déterminer l'antigène dans l'échantillon en se basant sur le degré d'augmentation de l'agglutination provoquée par la réaction antigène-anticorps, ou
(v) une étape, dans le cas où un anticorps est contenu dans l'échantillon, qui consiste à permettre à l'anticorps contenu dans l'échantillon et à l'anticorps fixé sur les microparticules, constituant le réactif de détermination, d'être en compétition l'un avec l'autre pour une réaction antigène-anticorps avec l'antigène constituant le réactif de détermination, et à déterminer l'anticorps dans l'échantillon en se basant sur le degré de diminution de l'agglutination provoquée par la réaction antigène-anticorps.

4. Procédé selon l'une quelconque des revendications 1 à 3, dans lequel l'antigène dans l'échantillon est **caractérisé en ce qu'**il est généralement présent dans des échantillons biologiques dérivés d'êtres humains ou d'animaux normaux mais est absent dans des échantillons biologiques dérivés d'un nombre extrêmement limité d'êtres humains ou d'animaux, et que ces êtres humains ou animaux possèdent un anticorps dirigé contre l'antigène.

5. Procédé selon l'une quelconque des revendications 1 à 4, dans lequel l'antigène dans l'échantillon est une IgA et l'anticorps dans l'échantillon est un anticorps anti-IgA.

6. Procédé selon l'une quelconque des revendications 1, 2, 4 et 5, qui est mis en oeuvre sans dilution de l'échantillon, en utilisant un réactif de détermination comprenant un anticorps et un antigène fixés sur des microparticules, afin d'éviter le phénomène de prozone.
